# EUROPÄISCHE PATENTSCHRIFT

(11) **EP 1 716 845 B1**
(45) Veröffentlichungstag und Bekanntmachung des Hinweises auf die Patenterteilung: **03.03.2010**
(21) Anmeldenummer: 06113022.5
(22) Anmeldetag: 25.04.2006
(51) Int. Cl.: A61K 9/06, A61K 31/13, A61K 31/195, A61K 47/08, A61K 47/10, A61K 31/57, A61K 31/137

(54) **Formulierung zur dermalen Anwendung**
Formulation for dermal application
Formulation pour l'application dermique

(30) Priorität: 26.04.2005 DE 102005019628
(43) Veröffentlichungstag der Anmeldung: 02.11.2006
(73) Patentinhaber: Technische Universität Braunschweig, 38106 Braunschweig (DE)
(72) Erfinder: Müller-Goymann, Christel C., 38104, Braunschweig (DE); Grüning, Nadja, 38106, Braunschweig (DE)
(74) Vertreter: Taruttis, Stefan Georg

(56) Entgegenhaltungen:
- EP-B- 0 215 423
- WO-A-2005/004981
- WINKLER A ET AL: "The influence of topical formulations on the permeation of 5-aminolevulinic acid and its n-butyl ester through excised human stratum corneum" EUROPEAN JOURNAL OF PHARMACEUTICS AND BIOPHARMACEUTICS, ELSEVIER SCIENCE PUBLISHERS B.V., AMSTERDAM, NL, Bd. 60, Nr. 3, 11. April 2005 (2005-04-11), Seiten 427-437, XP004967325 ISSN: 0939-6411

## Beschreibung

Die vorliegende Erfindung betrifft Formulierungen, die zur Penetrationsverstärkung amphiphiler, zwitterionischer, polarer oder lipophiler pharmazeutischer Wirkstoffe in der dermalen und/oder transdermalen Applikation geeignet sind.

Die dermale Applikation betrifft insbesondere eine Formulierung für den Wirkstoff 5-Aminolävulinsäure als Schlüsselsubstanz der Photodynamischen Therapie (PDT), bei der die Bildung hoch reaktiver Sauerstoffspezies, die von Porphyrinsystemen katalysiert wird, beispielsweise Singulett-Sauerstoff, Peroxidradikalen, Hydroxylradikalen und Superoxidradikal-Anionen sowie Wasserstoffperoxid mittels der als Licht eingestrahlten Energie ausgenutzt wird.

Medizinische Indikationen für die erfmdungsgemäße Formulierung von 5-Aminolävulinsäure (5-ALA) sind daher insbesondere aktinische Keratosen als Bestandteil der Photodynamischen Therapie, bei der Licht im Bereich von ca. 630 nm lokal oder laser-fokussiert im Anschluß an die Applikation eingestrahlt wird, sowie Akne.

### Stand der Technik

Die photodynamische Tumortherapie verwendet Photosensibilisatoren, die vorzugsweise eine hohe Tumorselektivität, geringe Zytotoxizität, kurze Verweildauer im Gewebe, hohe Ausbeute an Singulett-Sauerstoff und eine ausreichende Photostabilität aufweisen. Dies sind insbesondere Photosensibilisatoren, die sich vornehmlich in Tumorgewebe anreichern, so dass dieses bei der lokalen Bestrahlung direkt geschädigt wird. Als Photosensibilisatoren werden porphyrinhaltige Verbindungen oder deren Vorstufen in der Biosynthese eingesetzt, wie beispielsweise 5-Aminolävulinsäure oder deren Derivate.

So werden zur Behandlung nicht-kleinzelliger Bronchialkarzinome des Ösophaguskarzinoms und von Hamblasenkarzinomen oligomere Hämatoporphyrinether eingesetzt. Die Aktivierung erfolgt durch Laserbestrahlung bei einer Wellenlänge von 630 nm. Zur Behandlung von Alopezie wird beispielsweise Hämatoporphyrin eingesetzt, das bei vorsichtiger Abspaltung des Eisens aus Häm entsteht.

Als Alternative zu Porphyrinsystemen wird 5-Aminolävulinsäure verwendet, die in der Zelle biosynthetisch zu Protoporphyrin IX umgesetzt wird, welches durch Einlagerung von Fe²⁺ zu Häm wird. Ein pharmazeutisches Präparat, Metvix®, verwendet den Methylester der 5-Aminolävulinsäure aufgrund seines gegenüber der 5-Aminolävulinsäure lipophileren Charakters, wodurch die Permeation durch die Hautschichten erhöht ist. Dieses Derivat der 5-Aminolävulinsäure wird zur photodynamischen Therapie aktinischer Keratosen im Gesicht und am Kopf sowie von oberflächlichen und nodulären Basalzell-Karzinomen angewandt. Die applizierte 5-Aminolävulinsäure bzw. deren Methylester reichert sich in den Epithelzellen an und wird zu Protoporphyrin IX umgesetzt, das ein starker Photosensibilisator ist. Die Bestrahlung erfolgt bei einer Wellenlänge von 570 bis 670 nm. (Deutsche Apotheker Zeitung, Nr. 27, Seiten 3362 - 3368).

Die EP 0 215 423 B1 beschreibt transdermal resorbierbare Formulierungen von Arylpropionsäuren zu deren systemischer Verabreichung, die mit 1-15 Gew.-% Wirkstoff, 10-40 Gew.-% Poloxamer, 10-50 Gew.-% organischen Lösungsmittels und mindestens 10 Gew.-% Wasser enthalten. Als organische Lösungsmittel werden Ethanol, Isopropanol, 1,2-Propandiol, Glycerin, Isosorbid, Dimethylisosorbid, Polyoxyethylen(4)-laurylether, Polyoxypropylen-(15)-stearylether und DMSO genannt. Die Beispiele enthalten meist Ibuprofen, in zwei Fällen alternativ Ketoprofen oder Naproxen. Ein Gehalt an Lipiden wird nicht erwähnt.

Squillante et al. (European Journal of Pharmaceutics and Biopharmaceutics, 265-271 (1998)) beschreiben die Codiffusion von Propylenglycol und Dimethylisosorbid (DMIS) durch Maushaut in Gegenwart von Ölsäure, sowie die Permeation von Nifedipin aus einer Mischung, die neben der als Penetrationsverbesserer bekannten Ölsäure außerdem Propylenglycol und Dimethylisosorbid enthält. Dabei wird festgestellt, dass Ölsäure die Permeation von Dimethylisosorbid fördert, jedoch DMIS keine permeationsfördernde Wirkung für Propylenglycol und Ölsäure hat. Die Autoren postulieren auf diesem Befund der Codiffusion von Propylenglycol und DMIS einen Carrier-Mechanismus für den Arzneistoff Nifedipin, da dieser sowohl in Propylenglycol als auch in DMIS eine gute Löslichkeit aufweist. Über den bekannten Lösungsmitteleffekt von Propylenglycol und DMIS hinaus ist in der Veröffentlichung kein permeationsfördernder Effekt gezeigt.

Lee et al. (International Journal of Pharmaceutics, 105-114 (1997)) beschreiben eine Formulierung eines synthetischen Capsaicin-Analogs, die 20 bis 30 Gew.-% Poloxamer, 20 bis 40 Gew.-% Ethanol, 0 bis 20 Gew.-%, Propylenglycol und 5 Gew.-% Isopropylmyristat als Fettsäure in Wasser aufweist.

Die Rote Liste 2005 (Arzneimittelverzeichnis für Deutschland, Editio Cantor Verlag Aulendorf) offenbart unter der Bezeichnung Dolgit Mikrogel eine Zusammensetzung mit 5 Gew.-% Ibuprofen in 2-Propanol, Dimethylisosorbid, Poloxamer, mittelkettigen Triglyceriden, Lavendelöl und Pomeranzenblütenöl in Wasser.

Winkler und Müller-Goymann (European Journal of Pharmaceutics and Biopharmaceutics, 427-437 (2005)) vergleichen die Permeationverstärkung von Dolgit Mikrogel, Excipial Creme oder Basiscreme auf 5-Aminolävulinsäure und 5-Aminolävulinsäurebutylester. Unter diesen Formulierungen wies nur Dolgit Mikrogel einen Gehalt an Poloxamer auf. Beim Vergleich der Permeation von 5-Aminolävulinsäure und deren Butylester durch isoliertes menschliches Stratum corneum stellte sich heraus, dass die Permeation durch menschliche Haut am effektivsten von einer Formulierung des Butylesters in Dolgit Mikrogel (Ibuprofen-haltig) verstärkt wurde. Versuche mit modifizierten Formulierungen unter Verzicht auf den Wirkstoff Ibuprofen und/oder mittelkettige Triglyceride, sowie bei Variation der Anteile der übrigen Bestandteile eine Permeationsverbesserung des ALA-butylesters zu erreichen, schlugen fehl. In diesem Zusammenhang folgern Winkler und Müller-Goymann, dass der fluidisierende und permeationsverstärkende Effekt von Dolgit Mikrogel im Wesentlichen auf den Gehalt an Ibuprofensäure zurückgeht, während mittelkettigen Triglyceriden eine geringere permeationsverstärkende Wirkung zugeschrieben wird.

Zwischenzeitliche weitere Untersuchungen der Erfinder haben überdies gezeigt, dass die permeationsverstärkende Wirkung mittelkettiger Triglyceride für den 5-Aminolävulinsäurebutylester in Dolgit Mikrogel als bevorzugter Formulierung im Wesentlichen nur in Kombination mit einem Gehalt an Ibuprofen auftritt.

### Aufgabe der Erfindung

Vor dem bekannten Stand der Technik stellt sich der vorliegenden Erfindung die Aufgabe, eine Zusammensetzung bereitzustellen, mit der die Applikation bzw. der dermale Transport von 5-ALA so modifiziert bzw. verstärkt werden kann, dass in den Zielzellen erhöhte Konzentrationen von 5-ALA vorliegen.

Es ist ein weiteres Ziel der vorliegenden Erfindung, eine Zusammensetzung bereitzustellen, mit der der Transport, d.h. die Permeation von amphiphilen, zwitterionischen oder stark polaren Wirkstoffen durch das humane Stratum corneum verstärkt werden kann, besonders bevorzugt mit bei Raum- und/oder Körpertemperatur salbenähnlicher Konsistenz.

Für die medizinische Anwendung ist es von Bedeutung, dass die Formulierung für 5-Aminolävulinsäure bei Körpertemperatur eine hinreichend hohe Viskosität aufweist, so dass der Wirkstoff am Auftragungsort verbleibt und dort permeieren kann. Bei diesen Temperaturen der Anwendung dünnflüssige Formulierungen sind daher ungeeignet. Aufgrund der Instabilität von 5-Aminolävulinsäure (5-ALA) gegenüber Sauerstoff und damit auch gegenüber Luft ist es weiterhin wünschenswert, eine Salbengrundlage bereitzustellen, die separat, d.h. ohne den Wirkstoff hergestellt und gelagert werden kann, in die weiterhin der Wirkstoff, z.B. 5-Aminolävulinsäure auf einfache Weise homogen eingebracht werden kann.

Dabei soll die Formulierung noch die Permeation von 5-Aminolävulinsäure durch Epithel, beispielsweise menschliche Haut, fördern und, besonders bevorzugt, keine weiteren Wirkstoffe als die 5-Aminolävulinsäure enthalten.

Nach den Feststellungen in Vorversuchen, dass in bekannten Formulierungen die Konsistenz wesentlich durch Ibuprofen beeinflusst wird und dieser Wirkstoff nicht regelmäßig als Formulierungshilfe enthalten sein soll, stellt sich der vorliegenden Erfindung inbesondere die Aufgabe, eine an Ibuprofen freie Formulierung zur Verbesserung der Permeation amphiphiler, zwitterionischer, stark polarer oder lipophiler pharmazeutischer Wirkstoffe, insbesondere von 5-Aminolävulinsäure, bereitzustellen.

Eine weitere Aufgabe der vorliegenden Erfindung ist die Bereitstellung einer pharmazeutischen Zusammensetzung zur Behandlung aktinischer Keratosen und von Epithelzellkarzinomen, sowie Akne.

### Allgemeine Beschreibung der Erfindung

Die vorliegende Erfindung löst die oben gestellten Aufgaben durch Bereitstellen einer Formulierung, nach Anspruch 1, mit welcher pharmazeutische Wirkstoffe, insbesondere 5-ALA, in erhöhter Menge durch die menschliche Haut eingeschleust werden können. In der bevorzugten Ausführungsform ist die erfindungsgemäße Formulierung thermoreversibel gelierend, d.h. für die Zwecke der Erfindung, dass die Formulierung bei Körper- bis Raumtemperatur streichfähig bis fest ist, bei Temperaturen von ca. 12 °C eine niedrigere Viskosität aufweist, d.h. flüssiger ist. Die erfindungsgemäße Formulierung ist bei Temperaturen unterhalb der Körpertemperatur, beispielsweise von ca. 5 bis 12 °C lagerstabil und weist dann eine niedrige Viskosität auf, so dass ein Wirkstoff wie 5-Aminolävulinsäure mittels eines einfachen Rührers, beispielsweise eines Unguators^{®}, darin homogen verteilt werden kann. Die homogene Verteilung eines Wirkstoffs bei Raumtemperatur in die Formulierung ist mittels hochtouriger Rührwerkzeuge, beispielsweise eines Unguators^{®}, ebenfalls möglich. Bei der Temperatur der Applikation, d.h. etwa bei Körpertemperatur, weist die Formulierung unabhängig vom Gehalt an Wirkstoff, z.B. mit oder ohne 5-ALA, eine deutlich höhere Viskosität auf, nämlich eine salbenähnliche Konsistenz. Diese höhere Viskosität reicht aus, um den Wirkstoff für eine ausreichend lange Zeit (beispielsweise von 1 bis 5 h) zur Permeation auf der Haut zu halten, so dass die permeationsverstärkende Wirkung für den Wirkstoff am Applikationsort auftritt. Die erfindungsgemäße Formulierung weist eine im wesentlichen kolloidale Struktur auf.

Die erfindungsgemäße Formulierung weist keinen Gehalt an Ibuprofen auf, da dieser Wirkstoff nicht zur Einstellung der Viskosität oder Permeationseigenschaften erforderlich ist.

Weiterhin stellt die vorliegende Erfindung ein Herstellungsverfahren bereit, mit der die erfindungsgemäße Formulierung auf einfache Weise hergestellt werden kann, bevor schließlich der Wirkstoff zugegeben wird.

In einer bevorzugten Ausführungsform stellt die Erfindung eine Formulierung von 5-ALA oder einem anderen Wirkstoff bereit, mit der nach oberflächlicher Aufbringung der Formulierung eine erhöhte Konzentration des Wirkstoffs im Zielgewebe erhalten wird.

Die erfindungsgemäße Formulierung hat eine wässrige Grundlage, die
- 0, 1 - 20 Gew.-% zumindest eines amphiphilen, zwitterionischen oder stark polaren pharmazeutischen Wirkstoffs, vorzugsweise 5-Aminolävulinsäure,
- 10-30 Gew.-% eines Polyetherpolyols oder einer Mischung dieser, beispielsweise ausgewählt aus Polyethylenglycol (α-Hydro-ω-hydroxy-poly(oxy-1,2-ethandiol)), Polypropylenglycol und/oder Poloxamer (besonders bevorzugt Poloxamer 407, α-Hydro-ω-hydroxy-poly(oxyethylen)-poly(oxypropylen)-poly(polyoxyethylen)-blockcopolymer,
- 5-25 Gew.-% Dimethylisosorbid,
- 2,5-10 Gew.-% Triglyceride, vorzugsweise mittelkettige Triglyceride, insbesondere gemäß Deutschem Arzneibuch, und
- 5 - 20 Gew.-% eines C₂- bis C₄-Alkohols oder eine Mischung daraus, bevorzugt ausgewählt unter Ethanol und Isopropanol,
- Rest Wasser enthält,
- ohne Ibuprofen.

### Genaue Beschreibung der Erfindung

Die vorliegende Erfindung stellt eine Formulierung mit penetrationsverstärkender Wirkung für amphiphile, zwitterionische oder stark polare pharmazeutische Wirkstoffe bereit, die insbesondere zur topischen Aufbringung der pharmazeutischen Wirkstoffe auf Hautepithel geeignet ist, die bei Raumtemperatur fest bzw. halbfest ist und bei tieferen Temperaturen, beispielsweise bei 5 - 12 °C flüssig ist. Dieses Verhalten der temperaturabhängigen Viskositätsänderung wird für die Zwecke der Erfindung als thermoreversible Gelierung bezeichnet.

Die erfmdungsgemäße Formulierung stellt eine Matrix bereit, die amphiphile, zwitterionische und auch sehr polare Wirkstoffe integrieren kann und diese mit hoher Effektivität durch die menschliche Haut permeieren lassen kann, bevorzugterweise 5-Aminolävulinsäure.

Die erfindungsgemäße Formulierung weist ein Polyetherpolyol im Bereich von 10 - 30 Gew.-%, vorzugsweise 15 - 25 Gew.-%, besonders bevorzugt 20 Gew.-% auf. Das Polyetherpolyol kann unter Polyoxyethylenglycolen mit einem Molekulargewicht im Bereich von 1000 bis 30.000, Polyoxypropylenglycolen im Bereich von 1000 bis 30.000, Polyoxyethylenpolyoxypropylen-Blockcopolymeren mit einem Molekulargewicht im Bereich von 1000 bis 30.000 und Mischungen dieser ausgewählt werden. Als Polyoxyethylenglycol können auch Polyethylenglycole verwendet werden, bevorzugt beispielsweise Polyethylenglycol 600 (PEG - 12), Polyethylenglycol 400 (PEG - 8) und Polyethylenglycol 300 (PEG - 6).

Besonders bevorzugte Polyetherpolyole sind Poloxamere mit einem Molekulargewicht im Bereich von ca. 2000 bis 20.000 mit einem Anteil an Oxyethylengruppen im Bereich von 40 bis 90 Gew.-%, beispielsweise Poloxamer 124, Poloxamer 188, Poloxamer 237 und Poloxamer 338, wie in Ph. Eur. definiert. Ein besonders bevorzugtes Polyetherpolyol ist Poloxamer 407.

Weiterhin enthält die erfindungsgemäße Formulierung Dimethylisosorbid im Bereich von 5 - 25 Gew.-%, vorzugsweise im Bereich von 10-20 Gew.-% oder bis 15 Gew.-%, besonders bevorzugt 12-13 Gew.-%.

Ein weiterer Bestandteil der erfindungsgemäßen Formulierung sind Lipide, ausgewählt unter mittelkettigen Triglyceriden, fetten Ölen, Paraffinöl, flüssigen Wachsen, Isopropylmyristat, Cetyloleat und Mischungen dieser im Bereich von 2,5 - 10 Gew.-%., bevorzugter 4 - 6 Gew.-%. Besonders bevorzugt sind mittelkettige Triglyceride, beispielsweise entsprechend der Definition des Europäischen Arzneibuchs (Ph. Eur., 4. Ausgabe, Grundwerk 2002), d.h. ein Gemisch von Triglyceriden gesättigter Fettsäuren, hauptsächlich Caprylsäure und Caprinsäure, die zu mindestens 95 % gesättigte Fettsäuren mit 8 bis 10 Kohlenstoffatomen aufweisen. Besonders bevorzugt ist eine Fettsäurefraktion folgender Zusammensetzung: Capronsäure zu maximal 2 %, Caprylsäure zu 50 bis 80 %, Caprinsäure zu 20 bis 50 %, Laurinsäure zu höchstens 3% und Myristinsäure zu höchstens 1 %.

Weiter enthält die erfindungsgemäße Formulierung ca. 5-20 Gew.-%, bevorzugt ca. 10 - 15 Gew.-%, besonders bevorzugt ca. 12 - 13 Gew.-% eines C₂- bis C₄-Alkohols, vorzugsweise Ethanol, Propanol, Butanol, tert. Butanol, besonders bevorzugt Isopropanol.

Die erfindungsgemäße Zusammensetzung enthält weiterhin ca. 40 - 60 Gew.-% Wasser, bevorzugt ca. 45 - 55 Gew.-% Wasser, besonders bevorzugt ca. 50 Gew.-% Wasser. In die erfmdungsgemäße Formulierung können als Wirkstoffe insbesondere amphiphile, zwitterionische und stark polare Wirkstoffe integriert sein. Ein erfindungsgemäß bevorzugter Wirkstoff ist die 5-Aminolävulinsäure in einem Anteil von ca. 0, 1 - 20 Gew.-%, besonders bevorzugt ca. 5 - 15 Gew.-%, am bevorzugtesten ca. 10 Gew.-% an der Gesamtzusammensetzung.

Das erfmdungsgemäße Herstellungsverfahren beruht auf der Mischung der Formulierungsbestandteile, wobei vorteilhafterweise keine Vorbehandlungschritte einzelner Bestandteile, beispielsweise Erwärmung zur Verflüssigung, erforderlich sind.
So kann das Polyetherpolyol als Feststoff vorgelegt werden, zu dem dann Dimethylisosorbid (flüssig) zugegeben wird und anschließend das Lipid, beispielsweise mittelkettige Triglyceride (flüssig). Anschließend wird der C₂- bis C₄-Alkohol (flüssig) zugegeben, schließlich Wasser. Durch einfaches mechanisches Mischen bei Raumtemperatur, beispielsweise Rühren mit einem mechanischen hochtourigen Rührer, z.B. einem Unguator®, kann die erfindungsgemäße Formulierung hergestellt werden, wobei die Mischung unmittelbar während des Rührvorgangs geliert.

Die gemischte, gelierte Zubereitung ist z.B. über Nacht oder länger bei Raumtemperatur lagerstabil. Wirkstoffe für verschiedene Indikationen können durch Rühren eingearbeitet werden: Beispielsweise 5-Aminolävulinsäure (zur PDT), Bamipinmaleat (Antihistaminikum), Clotrimazol, Terbinafin (beides Antimykotika), Acetylsalicylsäure (nichtsteroidales Antirheumatikum und Analgetikum) oder Hydrocortisonacetat (Antiphlogistikum).

Das erfindungsgemäße Herstellungsverfahren kommt vorteilhafterweise mit einem einfachen hochtourigen Rührwerk zum Herstellen der Mischung aus, beispielsweise einem Apotheken-üblichen Unguator, so dass die Formulierung auch ohne zusätzlichen Bedarf an Geräten in Apotheken-üblichen Mengen hergestellt werden kann. Das einfache Herstellungsverfahren der Erfindung bietet auch in der großtechnischen Produktion Vorteile, da keine Bestandteile besonders vorbehandelt werden müssen, beispielsweise Verflüssigungen durch Erwärmen entbehrlich sind.

Als Alternative kann das Herstellungsverfahren die Schritte des Zusammenfügens der Formulierungsbestandteile Polyetherpolyol, Dimethylisosorbid, C₂ - C₄-Alkohol und Wasser, also noch ohne Lipid oder Wirkstoff, in einem Behälter aufweisen und beispielsweise in einer Fantaschale durchgeführt werden. Bei Lagerung bei niedriger Temperatur, z.B. 4 °C über Nacht ergibt sich eine klare viskose Lösung. Dieser Lösung kann, vorzugsweise nach Erwärmen auf Raumtemperatur der Lipidanteil zugesetzt und durch Rühren eingearbeitet werden. Die Mischung wird trübe und bei weiterer Erwärmung, z.B. durch das Rühren selbst, verfestigt sich die Mischung zu einem thermoreversiblen Gel. In dieses Gel kann dann der Wirkstoff durch Rühren eingebracht werden.

Die erfmdungsgemäße Formulierung hat den besonderen Vorteil, dass amphiphile oder zwitterionische und auch stark polare Wirkstoffe unmittelbar eingearbeitet werden können, ohne dass eine Derivatisierung erforderlich ist, um eine Formulierung des Wirkstoffs zu erhalten, die zur Permeation durch Epithel geeignet ist. So kann beispielsweise 5-Aminolävulinsäure ohne Derivatisierung zu ihrem Methylester in die erfindungsgemäße thermoreversibel gelierende Formulierung eingearbeitet werden und permeiert nach topischer Applikation in für die Therapie ausreichend großer Menge durch die Haut.

Ein weiterer Vorteil der vorliegenden Erfindung ist, dass einige Wirkstoffe in kostengünstigerer chemischer Struktur eingesetzt werden können, die selbst nicht durch spezielle Derivatisierung für die Permeation durch Epithel angepasst worden ist. So kann beispielsweise die 5-Aminolävulinsäure als solche als Wirkstoff eingesetzt werden, die wesentlich preisgünstiger erhältlich ist, als ihr durch die Haut permeierender Methylester, nämlich etwa um den Faktor 10.

Durch die effektivere Permeation des Wirkstoffs in die Haut kann die Einwirkzeit verkürzt werden, bzw. die während gleicher Einwirkzeit in die Haut permeierten Wirkstoffkonzentrationen sind höher. So lässt sich für die photodynamische Behandlung eine kürzere Einwirkzeit der 5-ALA anwenden oder auf Grund der höheren Permeationsraten eine effektivere Reduktion von Zellen während der Bestrahlung erreichen.

Die erfmdungsgemäße Formulierung ist lagerfähig und kann daher vorteilhafterweise unabhängig von dem später einzubringenden Wirkstoff hergestellt und ohne Kühlung, z.B. oberhalb von 13 °C gelagert werden. Aufgrund der thermoreversiblen Gelierung der erfindungsgemäßen Formulierung ist die Einbringung von Wirkstoffen einfach handhabbar, da die Wirkstoffe durch einfaches mechanisches Rühren in die Formulierung zu integrieren sind. Falls gewünscht kann die Viskosität der erfmdungsgemäßen Formulierung durch Kühlen, beispielsweise auf Temperaturen von 5 - 12 °C erniedrigt werden. Durch Erwärmen der nach Einmischen eines Wirkstoffs wirkstoffhaltigen Formulierung auf Raumtemperatur wird die gewünschte Viskosität von cremeartig oder halbfest bis fest erreicht, ohne dass weitere Bearbeitungschritte erforderlich wären. Diese Viskositätserhöhung bei höheren Temperaturen ist unabhängig vom eingemischten Wirkstoff.

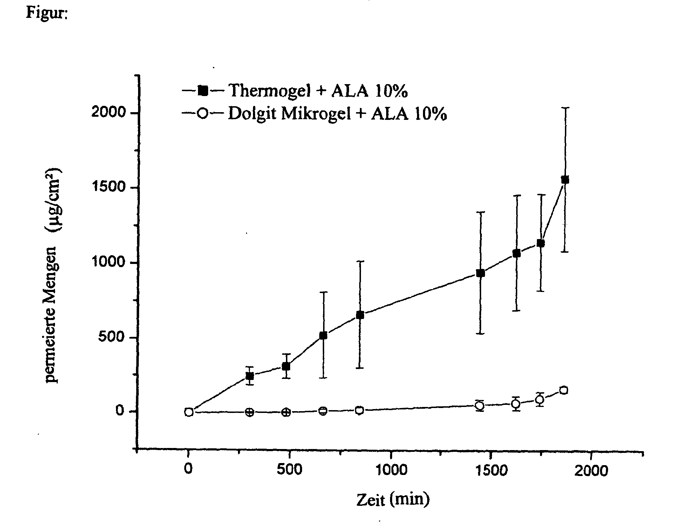

Bei der genauen Beschreibung der Erfindung wird als Beispiel für einen pharmazeutischen Wirkstoff 5-Aminolävulinsäure in der erfindungsgemäßen Formulierung eingesetzt. Dabei wird Bezug auf die Figur genommen, die die Permeation von 5-Aminolävulinsäure im erfindungsgemäßen Thermogel (■) und zum Vergleich in Dolgit Mikrogel (o) zeigt.

### Beispiel 1: Herstellung der erfindungsgemäßen Formulierung mit Poloxamer

In eine herkömmliche, Apotheken-übliche Unguatorkruke als Mischgefäß werden bei Raumtemperatur die folgenden Bestandteile eingewogen:
20,0 g Poloxamer 407 (Lutrol F 127, Bayer), 12,5 g Dimethylisosorbid (flüssig), 5,0 g mittelkettige Triglyceride (flüssig, entsprechend Europäischem Arzneibuch, Ph. Eur.), 12,5 g Isopropanol und schließlich 50,0 g Wasser.

Durch die Öffnung der Kruke wird ein mechanischer Rührer eingeführt und die Zusammenstellung durch Rühren gemischt. Die zunächst flüssige Mischung geliert unmittelbar während des Rührens bei ca. 1450 Upm für 1,5 min.

Die fertig gemischte, gelartige Zubereitung wird über Nacht bei Raumtemperatur gelagert. Der pH der Lösung bzw. der gelartigen Mischung lässt sich auf jeden gewünschten Wert einstellen.

### Beispiel 2: Formulierung mit Poloxamer und 5-Aminolävulinsäure als Wirkstoff

In eine nach Beispiel 1 hergestellte erfindungsgemäße Formulierung werden 10 Gew.-% 5-Aminolävulinsäure durch Rühren mit einem Unguator® eingearbeitet.

### Beispiel 3: Messung der Permeation von Wirkstoff aus der erfindungsgemäßen Formulierung durch humanes Stratum corneum

Für die Bestimmung der Permeation eines Wirkstoffs aus der erfindungsgemäßen Formulierung im Vergleich mit herkömmlichen wirkstoffhaltigen Formulierungen wurden 5-Aminolävulinsäure-haltige pharmazeutische Zubereitungen *in vitro* an isolierter menschlicher Haut getestet.

Als isolierte menschliche Haut wurde menschliches Stratum corneum verwendet, das aus Biopsien von plastischen Operationen stammte. Nach der Biopsie wurde subkutanes Fett entfernt und die Haut in Flüssigstickstoff eingefroren, dann bei -25 °C gelagert. Nach sanftem Auftauen wurde Stratum corneum durch Trypsinieren auf Filterpapier isoliert, das mit einer wässrigen 2% Trypsinlösung getränkt war. Nach einer Inkubation bei 37 °C für 24 h konnte Stratum corneum abgehoben werden, in einer 0,01-%igen wässrigen Lösung TrypsinInhibitor inkubiert und anschließend mehrere Male in Wasser gewaschen werden. Diese isolierte Hornschicht wurde getrocknet und bei Raumtemperatur im Exsikkator gelagert. Für Permeationsuntersuchungen wurde eine modifizierte Franzsche Zelle verwendet, deren Kompartimente durch das Stratum corneum abgetrennt wurden. Die Vorbehandlung des Stratum corneums erfolgt durch vollständiges Hydrieren in Wasser und Platzieren auf einem Polycarbonatfilter (Isopor Membranfilter, Typ TMTP, 5,0 µm, Millipore) zur Erhöhung der mechanischen Stabilität. Das Donorkompartiment wurde mit der 5-Aminolävulinsäurehaltigen erfindungsgemäßen bzw. der Vergleichsformulierung befüllt, wobei 5-Aminolävulinsäure zu 10 Gew.-% in den Formulierungen enthalten war. Die Formulierung wurde entsprechend Beispiel 2 in der jeweiligen wirkstofffreien Zusammensetzung hergestellt.

Als erfmdungsgemäße Zubereitung wurde das 5-Aminolävulinsäure-haltige Präparat, das nach Beispiel 2 erhältlich ist, im Vergleich zu einer Formulierung mit herkömmlicher Cremegrundlage gemäß Deutschem Arzneibuch (70 Teile Wasser, 30 Teile hydrophile Salbe aus 30 Teilen emulgierendem Cetylstearylalkohol (Typ A), 35 Teilen dickflüssigem Paraffin und 35 Teilen weißem Vaselin) und im Vergleich zu einer Formulierung in Dolgit Mikrogel (5 Gew.-% Ibuprofen, in 2-Propanol, Dimethylisosorbid, Poloxamer, mittelkettigen Triglyceriden, Lavendelöl, Pomeranzenblütenöl und Wasser) eingesetzt auf Permeation getestet.

Nach der Applikation wurde die durch die Haut permeierte Menge des Wirkstoffs gemessen. Gegenüber der herkömmlichen Cremegrundlage brachte die erfindungsgemäße Formulierung von 5-Aminolävulinsäure eine Erhöhung der Permeationsgeschwindigkeit durch das Stratum corneum um mindestens den Faktor 10 bis 20.

Das Permeationsverhalten der 5-Aminolävulinsäure in Poloxamer-haltigen Formulierungen ist in der beigefügten Figur gezeigt, die deutlich macht, dass die erfindungsgemäße Formulierung (■,Thermogel + 10% ALA) über die Messdauer eine deutlich stärkere Permeation durch das menschliche Stratum corneum ermöglicht, als die Formulierung Dolgit Mikrogel (o), wobei diese Formulierung den zusätzlichen Wirkstoff Ibuprofen enthält, der auch für die Konsistenz bei Raum- bzw. Körpertemperatur wesentlich ist. Der Stoffstrom an 5-ALA wurde für die erfindungsgemäße Formulierung zu 121 x 10⁻¹⁰ ± 23,9 x 10⁻¹⁰ g/cm²s bestimmt, der Permeationskoeffizient zu 937 x 10⁻¹⁰ ± 1,85 x 10⁻⁸ cms. Für die Vergleichszusammensetzung in Dolgit Mikrogel betrugen die Werte für den Stoffstrom 16,4 x 10⁻¹⁰ ± 3,69 x 10⁻¹⁰ g/cm²s bzw. den Permeationskoeffizienten 147,0 x 10⁻¹⁰ ± 33,0 x 10⁻¹⁰ g x cms.

Das erfindungsgemäße Thermogel führt zu einem permeierten Stoffstrom (Flux) des Wirkstoffs, hier beispielhaft durch 5-ALA dargestellt, der um den Faktor von ca. 7,5 größer ist, als nach dem bekannten Stand der Technik, nämlich aus einer Formulierung wie Dolgit Mikrogel.

Die Verbesserung der Permeation des Wirkstoffs aus der erfindungsgemäßen Formulierung ließ sich auch für weitere amphiphile, zwitterionische, stark polare oder lipophile Wirkstoffe feststellen, insbesondere für Bamipinmaleat, Clotrimazol, Terbinafin, Acetylsalicylsäure oder Hydrocortisonacetat. Diese Beobachtung wird derzeit darauf zurückgeführt, dass für die vorgenannten Wirkstoffe die Werte für die Lipophilie im Bereich derjenigen für 5-Aminolävulinsäure und deren Ester liegen. Denn entsprechend den log P-Werten aus Messungen des Oktanol-Wasser-Verteilungskoeffizienten (P), der zur Bestimmung der Lipophilie herangezogen wird, konnte auch für diese Wirkstoffe in der erfmdungsgemäßen Formulierung eine hohe Permeation durch das humane Stratum corneum bestimmt werden.

Der Lipophilie-Wert P gibt bei einem niedrigen Wert des Logarithmus von P (log P) eine niedrige Lipophilität an, ein hoher Wert des log P eine hohe Lipophilie. Der Verteilungskoeffizient P wird bei pH 7,4 und 21 °C in Oktanol-Wasser gemessen.

Für 5-ALA ist der Log P = -1,51692, für den 5-ALA-methylester = -0,94233, für den 5-ALAethylester = 0,84113, für den 5-ALA-butylester = 1,42315, für den 5-ALA-hexylester = 1,83883, für den 5-ALA-cyclohexylester = 1,49392 und für den 5-ALA-oktylester = 2,6199. Daher ist auch ein Log P von -1,15 für die Acetylsalicylsäure, von 2,18 für Hydrocortisonacetat, von 3,30 für Terbinafin und von 4,10 für Clotrimazol ein Hinweis darauf, dass Wirkstoffe mit Log P-Werten im Wasser-Oktanol-System bei pH 7,4, T = 21 °C im Bereich von -2 bis 5, vorzugsweise von -1,6 bis 4,5, eine geeignete Lipophilie aufweisen, um mit der erfindungsgemäßen Formulierung eine hohe Permeation durch die menschliche Haut zu erzielen.

## Patentansprüche

1. Pharmazeutische Formulierung, **gekennzeichnet dadurch, dass** sie
0,1-20 Gew.-% eines pharmazeutischen Wirkstoffs, ausgewählt unter 5-Aminolävulinsäure, Hydrocortisonacetat und Terbinafin,
10-30 Gew.-% Polyetherpolyol,
2,5-10 Gew.-% Lipide,
5-25 Gew.-% Dimethylisosorbid,
5-20 Gew.-% C₂ - C₄-Alkohol oder eine Mischung daraus,
40 - 60 Gew.-% Wasser
ohne Ibuprofen umfaßt.

2. Pharmazeutische Formulierung nach Anspruch 1, **dadurch kennzeichnet, dass** sie keine weiteren Inhaltsstoffe aufweist.

3. Pharmazeutische Formulierung nach einem der vorangehenden Ansprüche, **dadurch kennzeichnet, dass** das Polyetherpolyol Polyoxyethyleneinheiten und/oder Polyoxypropyleneinheiten aufweist.

4. Pharmazeutische Formulierung nach einem der vorangehenden Ansprüche, **dadurch kennzeichnet, dass** das Polyetherpolyol ausgewählt ist aus der Gruppe, die Polyethylenglycole, Polypropylenglycole und Poloxamere sowie Mischungen dieser, jeweils mit einem Molekulargewicht im Bereich von 1000 bis 30.000 umfaßt.

5. Pharmazeutische Formulierung nach einem der vorangehenden Ansprüche, **dadurch gekennzeichnet, dass** das Lipid ausgewählt ist aus der Gruppe, die mittelkettige Triglyceride, fette Öle, Paraffinöl, flüssige Wachse, Isopropylmyristat, Cetyloleat und Mischungen dieser enthält.

6. Pharmazeutische Formulierung nach einem der vorangehenden Ansprüche, **dadurch gekennzeichnet, dass** der C₂ - C₄-Alkohol Ethanol, Propanol, Butanol, tert. Butanol und/oder Isopropanol ist.

7. Pharmazeutische Formulierung nach einem der vorangehenden Ansprüche, **dadurch gekennzeichnet, dass** das Polyetherpolyol Poloxamer 407 ist.

8. Verfahren zur Herstellung einer pharmazeutischen Formulierung nach einem der vorangehenden Ansprüche.

## Claims

1. Pharmaceutical formulation, **characterized in that** it comprises
0,1 - 20% by weight of a pharmaceutical active compound, selected from 5-aminolevulinic acid, hydrocortisone acetate, and terbinafin,
10 - 30% by weight polyether polyol,
25-10% by weight lipids,
5 - 25% by weight dimethylisosorbide,
5 - 20% by weight C2- C4 alcohol or a mixture thereof,
40 - 60% by weight water,
without ibuprofen.

2. Pharmaceutical formulation according to claim 1, **characterized in that** it does not comprise any further ingredients.

3. Pharmaceutical formulation according to one of the preceding claims, **characterized in that** the polyetherpolyol has polyoxyethylene units and/or polyoxypropylene units.

4. Pharmaceutical formulation according to one of the preceding claims, **characterized in that** the polyetherpolyol is selected from the group which comprises polyethyleneglycol, polypropyleneglycol and poloxamers as well as mixtures thereof, each having a molecular weight in the region of 1000 to 30,000.

5. Pharmaceutical formulation according to one of the preceding claims, **characterized in that** the lipid is selected from the group which comprises medium chain triglycerides, fatty oils, paraffinic oil, fluid waxes, isopropylmyristate, cetyloleate and mixtures thereof.

6. Pharmaceutical formulation according to one of the preceding claims, **characterized in that** the C2- C4 alcohol is ethanol, propanol, butanol, tert. butanol and/or isopropanol.

7. Pharmaceutical formulation according to one of the preceding claims, **characterized in that** the polyetherpolyol is poloxamer 407.

8. Process for producing a pharmaceutical formulation according to one of the preceding claims.

## Revendications

1. Formulation pharmaceutique **caractérisée par le fait qu'**elle comprend
0,1 - 20 % en poids d'un agent actif, sélectionné parmi l'acide aminolévulinique-5, l'hydrocortisone acétate et la terbinafine,
10- 30 % en poids de polyétherpolyol,
2,5 - 10 % en poids d'un lipide,
5 - 25 % en poids de diméthylisosorbide,
5 - 20 % en poids d'alcool C₂ - C₄ ou un mélange de ces derniers,
40 - 60 % en poids d'eau
sans ibuprofène.

2. Formulation pharmaceutique selon la revendication 1, **caractérisée par** le fait de ne contenir aucun autre ingrédient.

3. Formulation pharmaceutique selon une des revendications précédentes, **caractérisée par le fait que** le polyétherpolyol présente des unités de polyoxyéthylène et/ou des unités de polyoxypropylène.

4. Formulation pharmaceutique selon une des revendications précédentes, **caractérisée par le fait que** le polyétherpolyol est sélectionné à partir du groupe comprenant le polyéthylène glycol, le polypropylène glycol et un poloxamère ainsi que des mélanges de ces derniers, chaque fois avec un poids moléculaire situé dans une plage allant de 1000 à 30.000.

5. Formulation pharmaceutique selon une des revendications précédentes, **caractérisée par le fait que** le lipide est sélectionné à partir du groupe contenant les triglycérides à chaînes moyennes, les huiles grasses, l'huile de paraffine, les cires liquides, l'isopropyle myristate, le cétyl oléate et des mélanges de ces derniers.

6. Formulation pharmaceutique selon une des revendications précédentes, **caractérisée par le fait que** l'alcool C₂ - C₄ est de l'éthanol, du propanol, du butanol, du butanol tertiaire et/ou de l'isopropanol.

7. Formulation pharmaceutique selon une des revendications précédentes, **caractérisée par le fait que** le polyétherpolyol est le poloxamère 407.

8. Processus destiné à préparer une formulation pharmaceutique d'après une des revendications précédentes.
